# EUROPEAN PATENT APPLICATION

(11) **EP 3 757 974 A1**
(43) Date of publication of application: **30.12.2020**
(21) Application number: 19183168.4
(22) Date of filing: 28.06.2019
(51) Int. Cl.: G09B 23/30, A61B 5/1495

(54) **A SIMULATOR DEVICE**

(71) Applicant: Stichting IMEC Nederland, 5656 AE Eindhoven (NL)
(72) Inventor: WIERINGA, Fokko, 3001 Leuven (BE); HERRANZ OLAZABAL, Jorge, 3001 Leuven (BE); VAN DEURSEN, Patrick, 3001 Leuven (BE)
(74) Representative: AWA Sweden AB

(57) **Abstract**

The present invention provides a simulator device (1) mimicking human tissue for calibrating a medical or non-medical device (2). The simulator device (1) comprises at least one optically active foil (3a-d) for dynamically varying optical tissue properties, at least one skin-mimicking area (4a-d) arranged on top of said at least one optically active foil (3a-d), wherein said skin-mimicking area (4a-d) is arranged for receiving said medical or non-medical device (2) during said calibration, and wherein said at least one optically active foil (3a-d) is further configured for absorbing and reflecting light emitted by said medical or non-medical device (2) during said calibration depending on a voltage applied to said optically active foil (3a-d). The simulator device (1) further comprises at least one optical feedback sensor (5a-d) for measuring the optical response of said at least one optically active foil (3a-d), and a control unit (6) configured for controlling the voltage applied to said at least one optically active foil (3a-d) and for varying the applied voltage dependent on information from said at least one optical feedback sensor (5a-d).

## Description

### Technical field

The present inventive concept relates to the field of patient simulators.

More particularly it relates to a simulator device for calibrating medical or non-medical devices, such as medical or non-medical devices that measure tissue optical properties (possibly in addition to non-optical physiological parameters) to derive medical parameters.

### Background

Pulse oximetry is a non-invasive test method that measures the oxygen saturation level (SpO2) in the blood, and allows for detecting how oxygen is being transported throughout the human body.

A pulse oximeter may be operated in transmissive mode, in which two or more wavelengths are transmitted through a body part, such as a fingertip or earlobe, towards a sensor. The absorbance and change in absorbance at each wavelength is measured, which is then related to the pulsating arterial blood. A pulse oximeter may also be of a reflective type, in which a photo detector is used adjacent to the emitter for detecting the diffuse backscatter of the light. Such pulse oximeters are not dependent upon thin transmissive body parts, and may e.g. be used on the forehead of a person.

Calibration of medical devices such as pulse oximeters is of course crucial to their function. For e.g. an invasive blood pressure sensor, one can easily provide a very accurately known pressure (both static and/or dynamic) as an external stimulus that will suffice to calibrate the pressure sensor. Such a straightforward approach is, however, not available for pulse oximeters as is also written into the pertaining international standard ISO 80601-2-61.

US 6 400 973 discloses an artificial blood flow simulator to be used to test or calibrate a pulse oximeter. The simulator has a body which is at least partially transparent to red and infrared light waves. Within the body is a light valve which is responsive to an electronic signal for varying the amount of light passing through the body. Connected to the light valve is a signal generator for generating a pulsating electronic signal which corresponds to a given blood flow.

EP 1 726 256 discloses a phantom device for mimicking anatomical structures, in which the imaging performance of an imaging apparatus may be tested and calibrated using predetermined dynamical behaviour.

There is however a need in the art for improved patient simulators that closely matches the actual static and dynamic optical properties of human tissue, thereby allowing for improved calibration of pulse oximeters.

### Summary

It is an object of the invention to at least partly overcome one or more limitations of the prior art. In particular, it is an object to provide a simulator device mimicking human tissue for calibrating a medical or non-medical device.

A further object of the invention is to provide a simulator device that closely matches the dynamic optical behaviour of tissue.

As a first aspect of the invention, there is provided a simulator device mimicking human tissue for calibrating a medical or non-medical device, said simulator device comprising
- at least one optically active foil for dynamically varying optical tissue properties,
- at least one skin-mimicking area arranged on top of said at least one optically active foil, wherein said skin-mimicking area is arranged for receiving said medical or non-medical device during said calibration, and wherein said at least one optically active foil is further configured for absorbing and reflecting light emitted by said medical or non-medical device during said calibration depending on a voltage applied to said optically active foil,
   and wherein said simulator device further comprises
- at least one optical feedback sensor for measuring the optical response of said at least one optically active foil, and
- a control unit configured for controlling the voltage applied to said at least one optically active foil and for varying the applied voltage dependent on information from said at least one optical feedback sensor.

The simulator device is for calibrating other devices. These devices may be either medical or non-medical devices. In embodiments, the simulator device is for calibrating medical devices, which may be an advantage in terms of quality and accuracy.

The medical or non-medical device may be a medical or non-medical device for transmitting light into tissue. As an example, the medical or non-medical device may be a pulse oximeter or any other medical or non-medical device configured for generating light emission into tissue and light detection from tissue. The medical or non-medical device may thus be a SpO₂ patient monitor or an Optical Coherence Tomography device.

The at least one optically active foil may be a single foil or a plurality of foils for dynamically varying optical tissue properties. Moreover, the at least one optically active foil may be electrically controllable optically active foils. The foil or foils may be arranged as a layer or a layer structure, such as forming a stack of layers. The optically active foil may comprise a liquid-crystal display (LCD), i.e. an optical device using the light-modulating properties of liquid crystals. There may thus be one LCD per optically active foil. The at least one optically active foil may be used for simulating different oxygen values by modifying the optical properties of the foil.

The at least one skin-mimicking area are arranged on top of the optically active layers. A skin-mimicking area has thus an area that is smaller than the area of the optically active foil. The skin-mimicking area may be a dyed structure, e.g. comprising natural chromophores such as melanin or artificial dyes to imitate a skin colour. As an example, skin-emulating materials like PVC, silicone and other materials known in the art of prothesis manufacturing may be used for making a visual skin appearance. These materials may be colored in various skin pigmentation strengths using chromophores that mimic the near infrared transmissive behavior of human skin pigment, such as iodine.

During calibration of the medical or non-medical device, the whole device or a probe of the device may be placed onto one or several of the skin-mimicking areas. Light emitted by the medical or non-medical device is thus transmitted through the skin-mimicking area to the optically active foil or foils. Depending on a voltage applied to the optically active foil, different wavelengths and intensities are reflected back to the medical or non-medical device.

Furthermore, the simulator device comprises an optical feedback sensor for measuring the optical response of the foil, e.g. the absorption of light. The optical feedback sensor may be for measuring the optical response of the foil for at least two wavelengths. The optical response may for example be absorption, transmission, scatter and/or diffuse reflection. The control unit is then configured to control the status of the optically active foil, i.e. configured to control that the applied voltage is actually producing desired absorption of the foil.

The inventors have found that there is a "memory-effect" in optically active foils which causes the optical properties of such materials to drift over time (while decreasing the dynamic range). Thus, once e.g. a DC voltage is applied to the foils, which are modifying the absorption of the red light, for a long time, the transparency of the LCD material is decreasing. This "memory effect" may even depend on the excitation history and "resting time" intervals. The above mentioned drift may also be frequency-dependent and stronger towards lower frequencies. This means that it may be difficult to induce a long-term stable dynamic photoplethysmographic waveform superimposed upon a freely programmable precise DC-offset, i.e. making it non-trivial to produce a very reproducible behaviour of voltage versus optical response (mixture of absorption, scatter and diffuse reflection).

The first aspect of the invention is based on the insight that including a feedback loop in terms of the at least one optical feedback sensor for measuring the optical response of said at least one optically active foil, and the control unit configured for controlling the voltage applied to the at least one optically active foil and further for varying the applied voltage dependent on information from said at least one optical feedback sensor, makes it possible to eliminate or at least decrease the risk of such a memory effect in the optically active foils. Another useful aspect is that the "memory effect" reverses when the foil is activated with reversed polarity. This can be exploited by reversing the polarity of foils that are used to create a periodic dip and recovery of oxygenation (e.g. simulated breathhold)

The simulator device of the first aspect thus makes it possible to closely match the dynamic optical behaviour of tissue, which e.g. causes the pulsating phenomenon called the photoplethysmographic pulse wave (PPG), is able to mimic both the geometry and the timing of photon paths through the tissue as closely as possible, thereby facilitating comparing the quality of ambient light rejection, movement artefact rejection. Further, the simulator device of the first aspect is advantageous over prior art devices, since such devices affect the photon timing and wavelength because they use a photodetector to sense the incoming light and provide an imitated light pulse by their own modulated light source.

The first aspect of the invention further provides a simulator device including few or no moving parts, i.e. a device that is extremely portable and stable over time. The simulator device may further very naturally mimic skin colour and thus allows to objectively evaluate and benchmark the influence of ambient light.

The voltage applied to the optically active foil(s) is performed by the control unit and is based on the output readings from the optical feedback sensor

The control unit may be wiredly connected to the optical feedback sensors and to the optically active foils. As an alternative, the control unit may be wirelessly connected to the optical feedback sensor and to the optically active foils.

The control unit is configured for analysing output data from the optical feedback sensor. Thus, the control unit may comprise computer program products configured for performing a method for comparing the output data from the optical feedback sensor with a reference value and vary the applied voltage based on the outcome of the comparison.

The control unit may comprise a processor and communication interface for communicating with optical feedback sensors and to the optically active foils and thus for receiving information at least from the optical feedback sensor.

The control unit may also be configured for controlling when to take measurements with the optical feedback sensor and when to vary the voltage applied to the optically active layers. Hence, the control unit may further comprise computer program products configured for sending operational requests to the optical feedback sensors and to the optically active foils. The operational requests may be based on analysis of received data from the optical feedback sensors or according to a pre-programmed operational scheme. For this purpose, the control unit may comprise a processing unit such as a central processing unit, which is configured to execute computer code instructions which for instance may be stored on a memory.

In embodiments of the first aspect, the simulator device comprises a plurality of optically active foils arranged in a stack. However, also passive layers may be incorporated into the stack.

Thus, the optically active foils may form a layered structure. The skin-mimicking areas may thus be arranged on top of the stack of optically active foils, i.e. on the outermost optically active foils in the stack.

The plurality of optically active foils may be arranged in a single stack or in a plurality of stacks.

At least one optically active foil in a stack may be configured to induce a spectral shift closely matching the spectral shift in tissue observed with varying concentrations of reduced haemoglobin, oxy-haemoglobin, carboxy-haemoglobin, methaemoglobin, bilirubin and/or other chromophores present in the blood stream and/or surrounding tissue.

In embodiments of the first aspect of the invention, the control unit is configured for applying a periodic voltage to said at least one optically active foil, thereby modulating the absorption of light in the optically active layer with oscillations.

The periodic voltage may thus give rise to a pulsation signal in one or several of the optically active foils. Such a foil may be configured for reflecting white light.

The periodic voltage may be applied to a foil that is configured for generating white light.

The control unit may be configured for applying a periodic voltage to a single optically active foil.

A pulsation signal may be used for simulating or mimicking a photoplethysmogram (PPG) signal, i.e. an optically obtained plethysmogram. A PPG signal may be used to detect blood volume changes a part of a person and may be obtained using a pulse oximeter.

The frequencies of heart beat and respiration cycle are free running oscillators, and such signals may be simulated via separate optical layers. The simulator device is thus able to include respiration-induced amplitude variations of the heart beat (PPG waves) with precise synchronization to the respiration cycle.

As an example, the uppermost optically active foil may be configured for generating a pulsation signal, such as a PPG signal. If for example the oxygen is set to a high value, the signal will still be visible because not all the red light is absorbed by the uppermost layer

Having a plurality of optically active layers facilitates the use of dynamic layers mimicking PPG and oxygen content in the blood. This information may be used to mimic skin wrinkle pattern and/or fingerprints and even body hair, e.g. in combination with known techniques used for prosthesis manufacturing.

As a further example, different optically active foils in the stack may be configured for absorbing different wavelengths of light.

As an example, the optically active foils may comprise one foil for absorbing wavelengths of between 600 and 700 nm, such as about 660 nm, and one foil for absorbing wavelengths between 900 and 1000 nm, such as about 940 nm.

Having optically active layers absorbing in 600-700 nm and 900-1000 nm, i.e. in visible red light and in infrared light, allows for simulation of peripheral capillary oxygen saturation, an estimate of the amount of oxygen in the blood (SpO₂).

As an example, the simulator device comprises a plurality of optically active foils and wherein the control unit is configured for applying a periodic voltage over at least one, such as a single, optically active foil of the plurality of active foils and further wherein the plurality of optically active foils comprises different optically active foils for absorption of different wavelengths.

The single optically active foil may be configured for reflecting white light, and the other optically active foils may thus be configured for absorption of different wavelengths, such as a green blue and yellow layer.

Thus, the white foil may be used for modulating the light like a heart pulsation (periodic AC component of PPG). The rest of the foils may be used to modulate the oxygen and to emulate the DC component of a PPG signal (due to venous flow, changes in oxygen and other tissue components).

As an example, the control unit may be configured for applying a periodic voltage to the uppermost optically active foil. In this way, when the simulated oxygen is set to a high value, the signals will still be visible because not all the red light is absorbed by the uppermost foil.

Furthermore, the simulator device may comprise one optical feedback sensor per layer of optically active foil. The optical feedback sensor may be configured to detect spectral transmission, reflection, scatter or a combination thereof.

Consequently, the simulator device may comprise a feedback loop that measures/controls the optical properties of each contributing optically active foil or layer of optically active foils if the foils are arranged in a stack. Thus, the control unit may be configured for controlling the voltage applied to all optically active foils and for varying the applied voltage on a foil dependent on information from the optical feedback sensor for measuring the optical response that optically active foil.

Having a feedback-loop per optically active layer is advantageous e.g. in that it allows for transferring an electrical PPG-waveform into a reproducible and very stable optical modulation of the simulator material that does not interfere with ambient light suppression techniques depending upon photon migration times through the tissue used by a vast majority of modern pulse oximetry devices.

The at least one skin-mimicking area may comprise several areas, forming a palette of different skin tones. As an example, the simulator device may comprise a single skin-mimicking area or at least two, such as at least three, such as at least five, skin-mimicking areas.

In embodiments of the first aspect, the simulator device comprises a plurality of skin-mimicking areas representing different skin tones. Every skin-mimicking area may thus be arranged on top of all the active layers and be parallel with the optically active foils.

As an example, at least two of the plurality of skin-mimicking areas may be arranged on top two different individually controlled stacks of optically active foils.

Thus, each skin-mimicking area may represent a different skin-tone, and each skin-mimicking area may be arranged either on top of a shared stack of optically active foils or be arranged on individual stacks of optically active foils, wherein the individual stacks may be configured to be individually controlled by the control unit. This may be advantageous in that the dynamic range mad be tailored for each skin type. Further, since the optically active foils may function as "leaky capacitors", and the surface area becomes smaller if there are several individual stacks of optically active foils, the response time may be faster.

In embodiments of the first aspect, the optical feedback sensor comprises at least one photo-detector, and wherein the control unit is configured for controlling that the voltage applied to said at least one optically active foil produces the desired spectral properties of the at least one optically active foil based on information from the at least one photo-detector and wherein the control unit is further configured for adjusting said applied voltage if the measured spectral properties are outside a targeted range.

The spectral properties may for example be absorption, transmission and/or scatter.

As an example, the spectral properties may be measured per optically active foil either at discrete wavelengths or in continuous wavelength spectra between 200 and 2700 nm.

As an example, the absorption of light may be measured at a first wavelength and a second wavelength, wherein the first wavelength is a red wavelength between 650 and 700 nm and said second wavelength is an infrared wavelength between 700 and 1000 nm.

Further, the control unit may be further configured for reversing the polarity of the voltage applied to the at least one optically active foil if the spectral properties of the at least one optically active foil is outside a targeted range, such as a targeted absorption range.

Thus, the control unit may be configured for first adjusting the voltage applied to an optically active foil and then, if the absorption of light of the optically active foil is not satisfactory, reverse the polarity.

Reversing the polarity may thus aid in decreasing the "memory effect" of the optically active foil. The inventors have found that the "memory effect" of the optically active foil may be very hard to predict straightforward and may co-depend on the "history of excitation", but that also reversing the polarity of the excitation voltage may aid in removing the "memory effect" of the optically active foil. The reversion in polarity may be performed at a predefined frequency for simulating a stable oxygen content value.

As an example, the control unit may be configured to reverse excitation voltage polarity between each cyclic signal (e.g. heartbeat and respiration) upon the moment of zero voltage crossing. In this way the dynamic range, linearity and repeatability of the response may be maximized for each cycle.

Furthermore, simulation of apnea, breath holds and the associated peripheral perfusion reflexes may require additional modulation of the optically active layers to simulate the shift in oxygen content. In order to produce very precise repeatable apnea, breath holds, downbreathing and the associated peripheral perfusion reflexes, the excitation voltage polarity for the these optically active layers may be reversed just before onset of a new cycle.

In embodiments of the first aspect, at least one optically active foil comprises a dielectric medium sandwiched between two conductive layers, and wherein the transparency of the dielectric medium is dependent on the charge of the capacitor formed by the dielectric medium and the two conductive layers.

The dielectric material may be an LCD material. The conductive layers may be transparent conductive layers, i.e. consist of a transparent material.

In embodiments of the first aspect, the simulator device further comprises an internal reference sensor arranged for transmitting light into the at least one optically active layer and for measuring the light that is reflected back to and/or transmitted to the internal reference sensor.

The internal reference sensor may function as an overall signal check loop, enabling fine tuning of the achieved overall simulation signals of the simulator device at the wavelengths applied by the medical or non-medical device under test.

The internal reference sensor may also be connected to the control unit. Thus, the control unit may be configured to control the response of the internal reference sensor and used that information for an overall status check.

The internal reference sensor may be configured for detecting the same physical parameters as the medical or non-medical device that is being tested or calibrated.

In embodiments of the first aspect, the control unit is further configured for synchronizing the voltage applied to the at least one active foil with an electrocardiogram (ECG) signal.

This allows for additional simulation of a synchronized electrocardiogram (ECG) signal as well-known in the art of ECG patient simulators. Thus, the control unit may be configured for receiving a simulated ECG signal or be configured for generating such a signal itself. For this purpose, the control unit may be arranged with a port for receiving such a simulated ECG signal.

However, the control unit may also be configured to calculate an ECG signal that would be measured between electrodes on a body, such as between electrodes that are attached at various positions on a body. As an example, the control unit may be configured to use internationally recognized data bases of 12-lead ECG signals and calculate an ECG signal that would be measured between electrodes at alternative locations on a body, such as between electrodes that are attached at various non-standard positions on a body.

The control unit may further be arranged to vary the time between an ECG wave and an applied periodic voltage, such as a periodic voltage simulating a PPG wave. As an example, the control unit may be configured to set a controlled delay time between an ECG signal and a periodic voltage, such as a PPG signal simulating a PPG wave.

The control unit may also be configured for transmitting the ECG signal to the medical or non-medical device being tested. Furthermore, the control unit may be configured to vary the time between an ECG R-wave and the PPG wave in a very precise and reproducible manner.

This may be advantageous since the travelling speed of the mechanical arterial Pulse Wave (which can be detected by PPG) is blood pressure dependent. The simulator device may thus facilitate the testing and development of non-invasive blood pressure monitoring without the need for frequent pressurizing a pneumatic cuff (which present blood pressure measurement devices require).

As a second aspect of the invention, there is provided a method for calibrating a medical or non-medical device comprising the steps of
a) providing a simulator device according to the first aspect and a medical or non-medical device to be calibrated;
b) arranging the medical or non-medical device on the at least one skin-mimicking area;
c) applying a voltage over at least one optically active foil;
d) transmitting light from the medical or non-medical device into the skin-mimicking area and the at least one optically active foil;
e) measuring the reflected light by said medical or non-medical device; and
f) calibrating said medical or non-medical device using information from the measured reflected light

This aspect may generally present the same or corresponding advantages as the former aspect. Effects and features of this second aspect are largely analogous to those described above in connection with the first aspect. Embodiments mentioned in relation to the first aspect are largely compatible with the second aspect.

As discussed in relation to the first aspect above, the method may be for calibrating devices that are either medical or non-medical devices. In embodiments, the method is for calibrating a medical device, which may be an advantage in terms of quality and accuracy.

Moreover, the medical or non-medical device may be a medical or non-medical device for transmitting light into tissue. As an example, the medical or non-medical device may be a pulse oximeter, an optical coherence tomography device, or any other medical or non-medical device configured for generating light pulses into tissue. The medical or non-medical device may thus be a SpO₂ patient monitor.

In embodiments of the second aspect, the medical or non-medical device is a pulse oximeter and wherein the reflected light is related to a simulated amount of oxygen in the blood.

Thus, the method may be used for calibrating a pulse oximeter using simulated amounts of oxygen levels in the optically active foil or foils of the simulator device.

Step b) comprises arranging the medical or non-medical device or a probe of the medical or non-medical device onto a skin-mimicking area of the simulator device.

Step c) may be initiated by the control unit of the medical or non-medical device. Step b) may comprise applying a voltage over each or some of the optically active foils if the simulator device comprises a plurality of optically active foils.

Step d) may comprise transmitting light of different wavelengths into the skin-mimicking area. As an example, step d) may comprise transmitting at least a first wavelength and a second wavelength, wherein the first wavelength is a red wavelength between 650 and 700 nm and said second wavelength is an infrared wavelength between 700 and 1000 nm. However, also a plurality of wavelengths may be transmitted in to the skin-mimicking area in step d).

Step e) is performed by the medical or non-medical device. For example, step e) may comprise calculating the ratio of the absorption of light in two know wavelengths, such as the first and second wavelengths mentioned above. As an example, step e) may comprise calculating the ratio of the absorption of emitted light of 660 nm and 940 nm based on measuring the reflected light.

Step f) may comprise relating the absorbance ratio with specific SpO₂ values or percentages. Step f) may comprise the formation of a look-up table, i.e. a table for relating an absorbance ratio to a specific SpO₂ value, such as a table relating the absorbance of two light frequencies, such as a red and an IR frequency, to a percentage of SpO₂.

In embodiments of the second aspect, step c) comprises applying a periodic voltage to at least one optically active foil, wherein said periodic voltage simulates a photoplethysmographic (PPG) pulse wave and/or a periodic respiration signal. Furthermore, step c) may comprise synchronizing the PPG pulse wave to an electrocardiogram (ECG) signal.

The ECG signal may be provided by an external unit, i.e. a unit not part of the simulator device. As an alternative, the ECG signal may be provided by the control unit of the simulator device. The ECG signal may for example be retrieved from an ECG-database that comprises well-classified heart arrhythmias.

As a further example, the method may comprise varying the time between the PPG pulse wave and the ECG R-wave of the ECG signal.

The control unit may also be configured to calculate an ECG signal that would be measured between electrodes that are attached at various positions on the body. Thus, in embodiments of the second aspect, the ECG signal is a simulated signal from electrodes positioned on alternative positions on a body.

### Brief description of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.
Fig. 1 is a schematic illustration of an embodiment of a simulator device according to the present disclosure.
Fig. 2 is a section view of an embodiment of a simulator device according to the present disclosure.
Fig. 3 is a section view of an embodiment of an optically active foil.
Fig. 4 is a schematic illustration of the process step of a method of the present disclosure.

### Detailed description

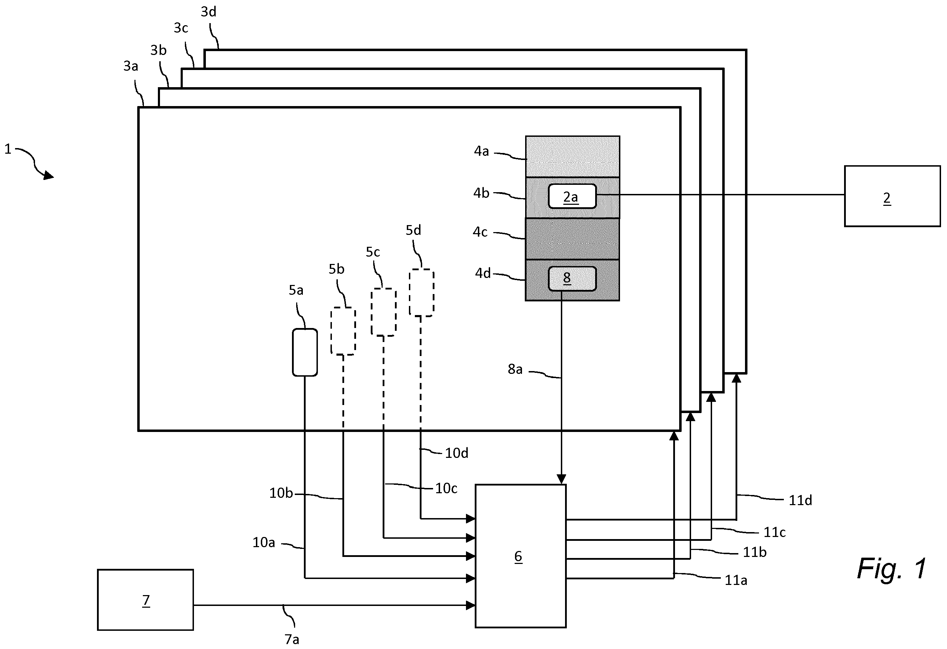

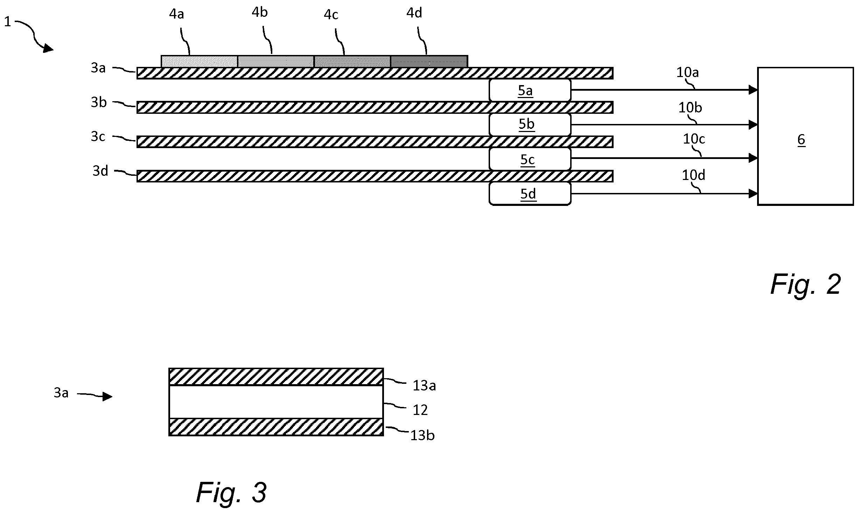

Fig. 1, and also Fig. 2, show an overall schematic embodiment of a simulator device 1 of the present disclosure. The simulator device 1 is for calibrating a medical or non-medical device 2, which for example be a pulse oximeter or a PPG device. The medical or non-medical device comprises in this example an SpO₂ probe 2a for measuring the oxygen content in blood. The simulator device comprises a stack of optically active foils 3a-d. In this example, there are four foils 3a-d, but the simulator device 1 could comprise a single foil or at least 5 foils, such as at least ten optically active foils. On the uppermost optically active foil 3a, there are four skin-mimicking areas 4a-4d, each area representing a different skin tone. It is also to be understood that the simulator device 1 could comprise any number of skin-mimicking areas, such as at least five, such as at least ten skin-mimicking areas. As indicated in Fig. 1, the SpO₂ probe 2a is placed on top of one or several of the skin-mimicking areas 4a-d during calibration.

The optically active foils 3a-d are used for e.g. simulating an oxygen content in the blood. To do this, the optically active foils 3a-d are configured for absorbing and reflecting light emitted by the SpO₂ probe 2a during the calibration. The amount of absorption (and reflection) is controlled by a voltage applied every optically active foil by a control unit 6. An optically active foil may thus comprise an LCD having a transparency dependent on the voltage applied over the LCD.

As indicated by arrows 11a-11c in Fig. 1, the control unit 6 is configured to control the voltage applied to each optically active foil independently. Thus, a first voltage may be applied to the uppermost foil 3a, a second voltage may be applied to the optically active foil 3b etc.

Furthermore, the simulator device comprises an optical feedback sensor 5a-d per optically active foil. Thus, there is one optical feedback sensor per optically active foil. This is further illustrated in the section view of Fig. 2. The feedback sensors 5a-d could thus be arranged within the stack of optically active foils 3a-d.

The optical feedback sensors 5a-d comprises in this example at least one photo-detector. The feedback sensors 5a-d are configured for measuring the optical response of each foil, i.e. feedback sensor 5a is configured for measuring the optical response of foil 3a, feedback sensor 5b is configured for measuring the optical response of foil 3b etc. The feedback sensors 5a-d and the control unit 6 are further configured for communicating with each other such that information of the optical response of each foil 3a-c is sent to control unit 6, as indicated by arrows 10a-d in Fig. 1. The control unit 6 is in this example wiredly connected to the feedback sensors 5a-d, but the wirelessly connected and to the feedback sensors 5a-d, e.g. if the feedback sensors 5a-d and the control unit 6 are connected to the same wireless communication network. The control unit is further configured to control the voltage applied to optically active foils 3a-d and for varying the applied voltage dependent on information from the optical feedback sensors 5a-d.

In this way, the "memory effect" of the LCD: s of the optically active foils may be reduced, i.e. the feedback control using the feedback sensors 5a-d and the control unit 6 allows for continuously controlling that the optically active foils functions properly. Consequently, the control unit 6 is further configured for controlling that the voltage applied to each optically active foil 3a-d produces the desired absorption of light each optically active foil 3a-dbased on information on the measured absorption of light from the photo-detectors of the feedback sensors 5a-d. The measured absorption of light may then be compared to e.g. reference values, such as predefined reference values, and the control unit may be configured for adjusting an applied voltage if the measured absorption of light is outside a target range, or e.g. above or below a reference value. Hence, the control unit 6 may comprise a communication interface such as a transmitter/receiver, via which it may receive data from the feedback sensors. The control unit 7 is thus configured for receiving information from the feedback sensors 5a-d and for sending control voltages via lines 11a-d based on the received data.

The control unit 7 is further configured to carry out a method for assessing if an optical response of an optically active foil 3a-d is satisfactory, such as within a reference target range. For this purpose, the control unit 7 may comprise a device having processing capability in the form of processing unit, such as a central processing unit, which is configured to execute computer code instructions which for instance may be stored on a memory. The memory may thus form a computer-readable storage medium for storing such computer code instructions. The processing unit may alternatively be in the form of a hardware component, such as an application specific integrated circuit, a field-programmable gate array or the like.

The control unit 6 may also be configured for controlling when to take measurements with the optical feedback sensors, i.e. if measurements are to be taken continuously or at discrete time points. Thus, the control unit 6 may further be configured for controlling the initiation of the measuring op optical response using the feedback sensors 5a-d. For this purpose, the processing unit of the control unit 7 may further comprise computer code instructions for sending operational requests to the optical feedback sensors 5a-d.

The system 1 may further comprise display means connected to the control unit 6 for displaying on a screen one or several optical responses measured by the feedback sensors 5a-d.

As an alternative, the control unit 7 may be configured just for receiving the data from the feedback sensors 5a-d. This data may then be sent to an external unit for further processing. As an example, the data may be transmitted to a storage unit (not shown), which may be a disk drive of a computer. A communication interface of the control unit 6 may thus be configured to transmit received data from feedback sensors 5a-d to a remote storage unit, such as a cloud-based storage unit. A remote software may then be used for assessing the optical response of the optically active foils 3a-d. Consequently, the data received by the control unit 6 may be sent to a computer, and such a computer may have a central processing unit (CPU) and may further be provided with a software for causing the CPU to perform operations so as to determine if the optical response of the foils 3a-d are satisfactory, such as within a certain target range.

The control unit 1 is in this example further configured for reversing the polarity of the voltage applied to the optically active foils 3a-d if the desired absorption of light in the optically active foils is not satisfactory, such as outside an absorption target range. This may aid in decreasing the risk of memory effect even further.

The foils 3a-d are configured for absorbing different wavelengths of light. As an example, one of the foils 3a-d may be configured for absorbing a first wavelength about 660 nm, whereas another foil may be configured for absorbing a second wavelength of about 940 nm.

Furthermore, the control unit 6 is in this example configured for applying a periodic voltage to one of the optically active foils 3a-d, thereby modulating the absorption of light in the optically active layer with oscillations. As an example, the control unit 6 may be configured to provide a periodic voltage to the uppermost foil 3a.

The absorption properties of the foils 3a-d make it possible to simulate different oxygenation in blood vessels, and the oscillating behaviour pf the uppermost foil makes it possible to simulated dynamic variations in the absorption, such as variations depending on heart rate etc. Thus, the simulator device1 is able to include respiration-induced amplitude variations of the heart beat (PPG waves) with precise synchronization to the respiration cycle.

Furthermore, the earth may be common to all the optically active layers. It may be advantageous to have the foil, which is producing the PPG signals (white), being the most external one 3a. In this way, the earth will always be the most external layer and the electrical influence of the foils 3a-d into the probe will be greatly attenuated.

The medical or non-medical device 2 comprising the SpO2-probe 2a may determine an SpO₂ value by calculating the ratio of the absorption of light in two know wavelengths, such as 660 nm and 940 nm. To calculate this ratio, the pulsation of the light intensity due to the modulation of the blood volume using the periodic voltage may be a key factor.

To decrease the value of the calculated oxygen, the absorption of red light may be decreased in comparison to the IR light. Therefore, the light intensity of the red light, will decrease when the SpO₂ is increasing.

Consequently, during calibration, the probe 2a of the medical or non-medical device 2 under test (pulse oximeter or multiparameter monitor) is placed upon the chosen skin type and collects the simulated optical signals.

As also seen in Fig. 1, the simulator device 1 comprises an internal reference sensor 8 arranged for transmitting light into the optically active layers 3a-d and for measuring the light that is reflected back to the internal reference sensor 8. This information is also sent to control unit 6 as indicted by arrow 8a in Fig. 1. This internal reference sensor 8 allows for (more input on why internal reference sensor 8 is used). The reference sensor 8 could be arranged on top one of the skin-mimicking areas 4a-d, or it could be arranged within any of the optically active foils 3a-d.

There is further an ECG unit 7 configured for simulating an ECG (electrocardiography) signal and for sending this to the control unit 6, as indicated by arrow 7a in Fig. 1. The control unit 7 is then configured for synchronizing the voltage applied to the optically active foils 3a-d with the received ECG signal.

The ECG unit 1 may be part of the simulator device 1 or be an external unit.

Furthermore, the control unit may also comprise software that generates a photoplethysmographic (PPG) waveform plus synchronized ECG at a chosen heart rate. As an alternative, the control unit 6 may be configured to receive a signal from e.g. a computer comprising a control software for generating generates a photoplethysmographic (PPG) waveform plus synchronized ECG at a chosen heart rate. The simulator device 1 is thus capable of being synchronized to an electrical electrocardiogram (ECG) signal as well-known in the art of ECG patient simulators. Furthermore, the simulator device 1 allows to vary the time between an ECG R-wave and the PPG wave in a very precise and reproducible manner. The travelling speed of the mechanical arterial Pulse Wave (which can be detected by PPG) is blood pressure dependent. The simulator device 1 may thus aid in developing non-invasive blood pressure monitoring without the need for frequent pressurizing a pneumatic cuff (which present blood pressure measurement devices require).

Fig. 3 is a schematic section view of one of the optically active foils 3a. The foils 3a comprises a dielectric medium12 sandwiched between two conductive layers 13a and 13b. The transparency of the dielectric medium 12 is dependent on the charge of the capacitor formed by the dielectric medium 12 and the two conductive layers 13a and 13b, i.e. on the voltage applied over the foil 3a.

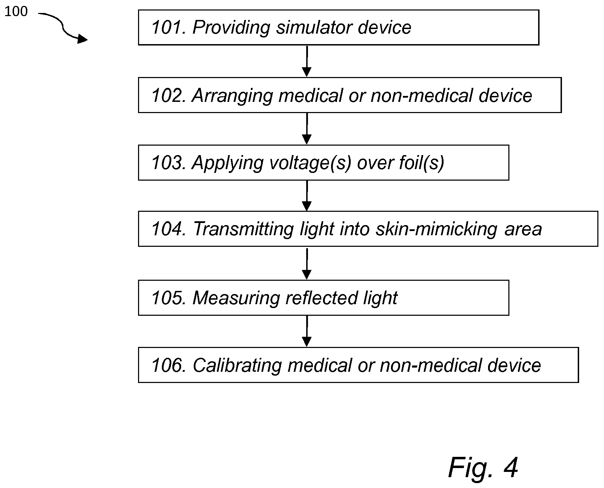

Fig. 4 schematically illustrates a method 100 for calibrating a medical or non-medical device 2 using the simulator device of the present disclosure. The method comprises the steps of
a) providing 101 a simulator device 1 as disclosed herein and a medical or non-medical device 2 to be calibrated;
b) arranging 102 the medical or non-medical device 2 on at least one skin-mimicking area 4a-d;
c) applying 103 a voltage over at least one optically active foil 3a-d;
d) transmitting 104 light from the medical or non-medical device 2 into the skin-mimicking area 4a-d and the at least one optically active foil 3a-d;
e) measuring 105 the reflected light by the medical or non-medical device 2; and
f) calibrating 106 the medical or non-medical device 2using information from the measured reflected light.

The medical or non-medical device 2 to be calibrated usually transmits light of two colours, 660 nm and 940 nm. Depending on the intensity of each detected wavelength, the device 2 is able to determine the amount of light absorbed of each colour. This absorption information can be processed and calibrated to give values of instantaneous arterial oxygen saturation. With the simulator device 1, the voltage of one active foil 1 is modulated with a periodic input signal to modulate the absorption with PPG like oscillations, since also a person's body onto which the medical or non-medical device is to be used pulsates (with PPG like signals). On top of that the voltage of the rest of optically active foils 3a are modulated such that the absorption of each wavelength is modulated. Ion this way, the simulator device 1 produces the same absorption the body produces with different values of oxygen.

As discussed above, the feedback sensors 5a-d is measuring continuously, such that the control unit 7 is continuously controlling that the applied voltage is actually producing the desired SpO₂ value, i.e. the control unit 7 controls that the voltage is producing the desired absorption for each light.

Thus, the method may also comprise a step of controlling that the voltage applied to the optically active foils (3a-d) produces the desired absorption of light in the at least one optically active foil (3a-d) based on information on the measured absorption of light from at least one photo-detector in a feedback sensor. The method may then comprise adjusting the applied voltage if the measured absorption of light is outside an absorption target range.

Furthermore, the method may comprise the step of reversing the polarity of the voltage applied to optically active foils 3a-d if the desired absorption of light in the optically active foils 3a-d is outside a target range, such as a reference interval.

Since the memory effect is quite unpredictable, it may be required to have a control on each of the optically active foils 3a-d individually and also the internal reference sensor 8. This internal reference sensor 8 may be a well calibrated sensor, with better signal-to-noise ratio than the sensor 2a of the medical or non-medical device 2.

The simulator device 1 may thus be a reflective simulator. It is an advantage of having a reflective simulator that does not receive and emit modulated light to emulate values of oxygen or PPG waveforms. The changes in the detected light by the medical or non-medical device 2 when calibrated in a reflective simulator are changes in absorption which leaves the timing and wavelengths of the photons unaltered. This in combination with the programmable ECG gives the possibility of testing Pulse arrival time, in an unprecedented way, e.g. to test the stability a medical or non-medical device 2 at different temperatures.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A simulator device (1) mimicking human tissue for calibrating a medical or non-medical device (2), said simulator device (1) comprising
- at least one optically active foil (3a-d) for dynamically varying optical tissue properties,
- at least one skin-mimicking area (4a-d) arranged on top of said at least one optically active foil (3a-d), wherein said skin-mimicking area (4a-d) is arranged for receiving said medical or non-medical device (2) during said calibration, and wherein said at least one optically active foil (3a-d) is further configured for absorbing and reflecting light emitted by said medical or non-medical device (2) during said calibration depending on a voltage applied to said optically active foil (3a-d),
and wherein said simulator device (1) further comprises
- at least one optical feedback sensor (5a-d) for measuring the optical response of said at least one optically active foil (3a-d), and
- a control unit (6) configured for controlling the voltage applied to said at least one optically active foil (3a-d) and for varying the applied voltage dependent on information from said at least one optical feedback sensor (5a-d).

2. A simulator device (1) according to claim 1, wherein the simulator device (1) comprises a plurality of optically active foils (3a-d) arranged in a stack.

3. A simulator device (1) according to claim 2, wherein different optically active foils (3a-d) are configured for absorbing different wavelengths of light.

4. A simulator device (1) according to claim 2 or 3, wherein the simulator device (1) comprises one optical feedback sensor (5a-d) per layer of optically active foil (3a-d).

5. A simulator device (1) according to any previous claim, wherein the simulator device (1) comprises a plurality of skin-mimicking areas (4a-d) representing different skin tones.

6. A simulator device (1) according to claim 5, wherein at least two of the plurality of skin-mimicking areas (4a-d) are arranged on top two different individually controlled stacks of optically active foils (3a-d).

7. A simulator device (1) according to any previous claim, wherein said optical feedback sensor (5a-d) comprises at least one photo-detector, and wherein the control unit (6) is configured for
- controlling that the voltage applied to said at least one optically active foil (3a-d) produces the desired spectral properties of the at least one optically active foil (3a-d) based on information from the at least one photo-detector and wherein the control unit (6) is further configured for adjusting said applied voltage if the measured spectral properties are outside a targeted range.

8. A simulator device (1) according to claim 7, wherein the control unit (6) is further configured for reversing the polarity of the voltage applied to the at least one optically active foil (3a-d) if the spectral properties of the at least one optically active foil (3a-d) is outside a targeted absorption range.

9. A simulator device (1) according to claim 7 or 8, wherein the spectral properties are measured per optically active foil (3a-d), either at discrete wavelengths or in continuous wavelength spectra between 200 and 2700 nm.

10. A simulator device (1) according to any previous claim, wherein the control unit (6) is configured for applying a periodic voltage to said at least one optically active foil (3a-d), thereby modulating the absorption of light in the optically active layer with oscillations.

11. A simulator device (1) according to claim 10, wherein the simulator device (1) comprises a plurality of optically active foils (3a-d) and wherein the control unit (6) is configured for applying a periodic voltage over at least one optically active foil of the plurality of active foils (3a-d) and further wherein the plurality of optically active foils (3a-d) comprises different optically active foils (3a-d) for absorption of different wavelengths.

12. A simulator device (1) according to any previous claim, wherein the at least one optically active foil (3a-d) comprises a dielectric medium sandwiched between two conductive layers, and wherein the transparency of the dielectric medium is dependent on the charge of the capacitor formed by the dielectric medium and the two conductive layers.

13. A simulator device (1) according to any previous claim, wherein the simulator device (1) further comprises an internal reference sensor (8) arranged for transmitting light into the at least one optically active layer and for measuring the light that is reflected back to and/or transmitted to the internal reference sensor (8).

14. A simulator device (1) according to any previous claim, wherein the control unit (6) is further configured for synchronizing the voltage applied to the at least one active foil (3a-d) with an electrocardiogram (ECG) signal.

15. A method for calibrating a medical or non-medical device (2) comprising the steps of
a) providing (101) a simulator device (1) according to any one of claims 1-14 and a medical or non-medical device (2) to be calibrated;
b) arranging (102) the medical or non-medical device (2) on the at least one skin-mimicking area (4a-d);
c) applying (103) a voltage over at least one optically active foil (3a-d);
d) transmitting (104) light from the medical or non-medical device (2) into the skin-mimicking area (4a-d) and the at least one optically active foil (3a-d);
e) measuring (105) the reflected light by said medical or non-medical device (2); and
f) calibrating (106) said medical or non-medical device (2) using information from the measured reflected light.
